# EUROPEAN PATENT SPECIFICATION

(11) **EP 3 737 671 B1**
(45) Date of publication and mention of the grant of the patent: **17.11.2021**
(21) Application number: 19705818.3
(22) Date of filing: 11.01.2019
(51) Int. Cl.: C07D 265/32, C07C 231/02, C07C 51/29, C07C 59/135, C07C 235/16

(54) **PROCESS FOR THE PREPARATION OF 4-(4-AMINOPHENYL)MORPHOLIN-3-ONE**
VERFAHREN ZUR HERSTELLUNG VON 4-(4-AMINOPHENYL)MORPHOLIN-3-ON
PROCÉDÉ DE PRÉPARATION DE 4-(4-AMINOPHÉNYL)MORPHOLIN-3-ONE

(30) Priority: 12.01.2018 HU 1800007
(43) Date of publication of application: 18.11.2020
(73) Proprietor: Richter Gedeon Nyrt., 1103 Budapest (HU)
(72) Inventor: SZABÓ, Tamás, 2510 Dorog (HU); NEU, József, 1133 Budapest (HU); GARADNAY, Sándor, 1037 Budapest (HU)
(74) Representative: Kutzenberger Wolff & Partner
(86) International application number: PCT/IB2019/050213
(87) International publication number: WO 2019/138362

(56) References cited:
- CN-A- 102 464 580
- CN-A- 104 098 525
- ANIL C MALI ET AL: "Facile approach for the synthesis of rivaroxaban using alternate synthon: reaction, crystallization and isolation in single pot to achieve desired yield, quality and crystal form", SUSTAINABLE CHEMICAL PROCESSES, vol. 48, no. 1, 13 July 2015 (2015-07-13), page 5900, XP055230109, DOI: 10.1186/s40508-015-0036-3

## Description

### FIELD OF THE INVENTION

The present invention provides a process suitable for industrial scale manufacture for the preparation of 4-(4-aminophenyl)morpholin-3-one of Formula (I), the key intermediate of rivaroxaban.

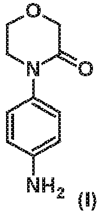

### BACKGROUND OF THE INVENTION

Rivaroxaban among others, is used for anticoagulant medication (blood thinner). It is commonly used to prevent blood clots. In the preparation of rivaroxaban the 4-(4-aminophenyl)morpholin-3-one **(I)** molecule is a frequently used key intermediate. The economy of industrial scale preparation of rivaroxaban highly depends on the appropriate preparation of this key intermediate.

Several industrially applicable procedures are available for the preparation of 4-(4-aminophenyl)morpholin-3-one **(I). Scheme 1** is based on the procedure applied in US2003153610 A1. 4-(4-aminophenyl)morpholin-3-one **(I)** is prepared from 4-nitro-fluorobenzene **(II)**, in the first step it is coupled with morpholin-2-one **(III)** in N-methylpyrrolidone, as a base sodium-hydride is used. The obtained 4-nitro-phenylmorpholinone **(IV)** is reduced in tetrahydrofuran with palladium on carbon catalyst at a temperature of 70°C. This method is not advisable when the reaction is performed at an industrial scale, the overall yield of the two steps is under 7%.

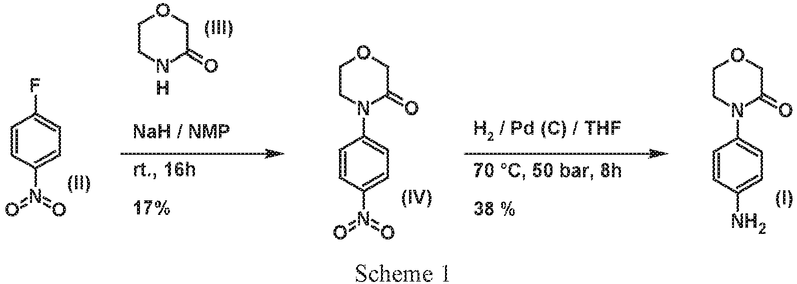

In patent application WO2005/026135 A1 (Bayer Healthcare AG) a more economic process is disclosed. In the synthesis according to **Scheme 2** N-(2-Hydroxyethyl)aniline **(V)** is reacted with chloroacetyl chloride and 4-phenylmorpholin-3-one **(VI)** is obtained. Subsequently, in a nitration reaction 4-(4-nitrophenyl)morpholin-3-one **(IV)** is obtained. The nitro compound **(IV)** is hydrogenated in ethanol at 80°C to obtain compound **(I)**. Although in this procedure the overall yield of compound **(I)** is 52%, in industrial scale the nitration procedure is problematic, requires special equipment, and in the respect of safety it is disadvantageous.

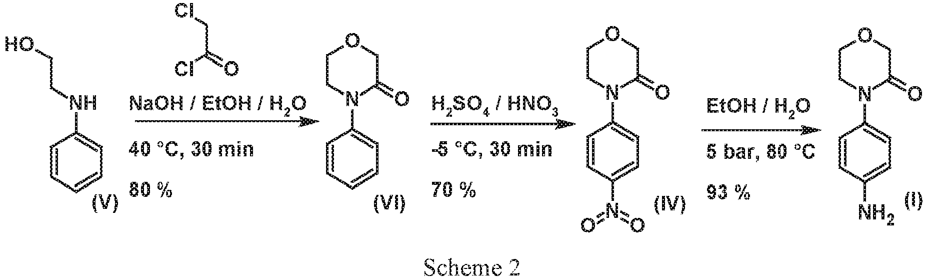

According to method of **Scheme 3** (Mederski et al.: Bioorganic & Medicinal Chemistry Letters 14, 2004 p. 5817―5822) 4-nitro-aniline **(VII)** is reacted with 2-(2-chloroethoxy)acetyl chloride **(VIII)** in toluene. The given 2-(2-chloroethoxy)-N-(4-nitrophenyl)acetamide **(IX)** is boiled with potassium-carbonate in acetonitrile to obtain the ring-closed 4-(4-nitrophenyl)morpholin-3-one **(IV)**, which is then reduced with palladium on carbon in methanol to obtain the target compound 4-(4-aminophenyl)morpholin-3-one **(I)**. Yield data are not revealed.

The 2-(2-chloroetoxy)acetyl chloride **(VIII)** used in this procedure is not an easily available compound. Acyl chlorides generally are produced "in-situ" from the appropriate carboxylic reagents. It is a common feature of these compounds that during forming of acyl chloride substantial quantity of hydrochloric acid evolves, that amortizes the equipment and accessories, burdens the environment, while the reaction low atom economy makes the process disadvantageous.

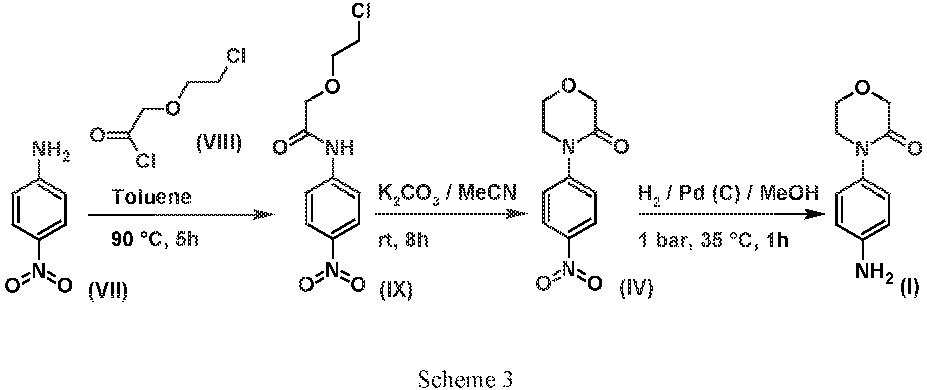

Mali et al. in (Sustain Chem. Process (2015) 3:11) according to the process of **Scheme 4** start from 2-(2-chloroethoxy)acetic acid **(X)**, with the aid of catalysis by boric acid 2-(2-chloroethoxy)-N-(4-nitrophenyl)acetamide **(IX)** is formed (yield: 50%), then in the presence of sodium-hydroxide base in phase-transfer conditions the closed-ring compound **(IV)** is obtained (yield 90%), then with hydrogenation the expected product **(I)** is obtained (yield 75%). The yield of the whole procedure is only 33.8%, not enough for an economic industrial application.

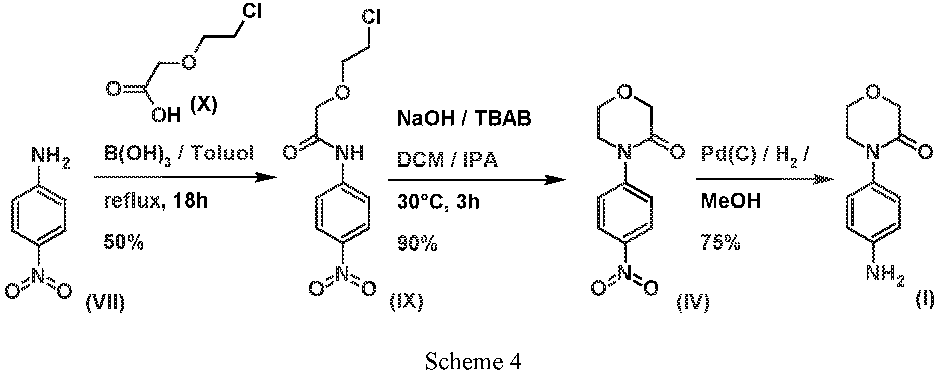

The former procedures do not deal with formation of 2-(2-chloroethoxy)acetic acid **(X),** however from the literature several such methods are known. In the procedure of EP1640373A1 the 2-(2-chloroethoxy)acetic acid **(X)** is formed by oxidation of 2-(2-chloroehtoxy)ethanol **(XI)**. Oxidation was carried out by hydrogen peroxide with a catalysis by sodium-tungstate.

It is a drawback of this process that to carry out the oxidation with 30% hydrogen peroxide at 90°C is dangerous, moreover the yield of 19% is far from the industrial expectations.

CN 104 098 525 A discloses the synthesis of 2-(2-chloroethoxy)acetic acid from 2-(2-chloroethoxy)ethanol. According to the process described in WO2008075152A1 the 2-(2-chloroethoxy)ethanol **(XI)** was oxidized with Jones reagent (yield: 80 %). It is disadvantageous that the chromium trioxide in the Jones reagent is extremely toxic for humans and the environment. Refuse disposal of the large amount side product Cr(III) and unreacted Cr(VI) after the reaction in precipitate form is a tedious and uneconomical. Moreover, the other component of Jones reagent, the 30-40 m/m% sulphuric acid, can promote formation of symmetric ester by-product.

The primary objective of this invention is to provide simple and cost effective industrially applicable process for the preparation of 4-(4-aminophenyl)morpholin-3-one, a rivaroxaban key intermediate.

### SUMMARY OF THE INVENTION

Our invention is summarized in **Scheme 5.**

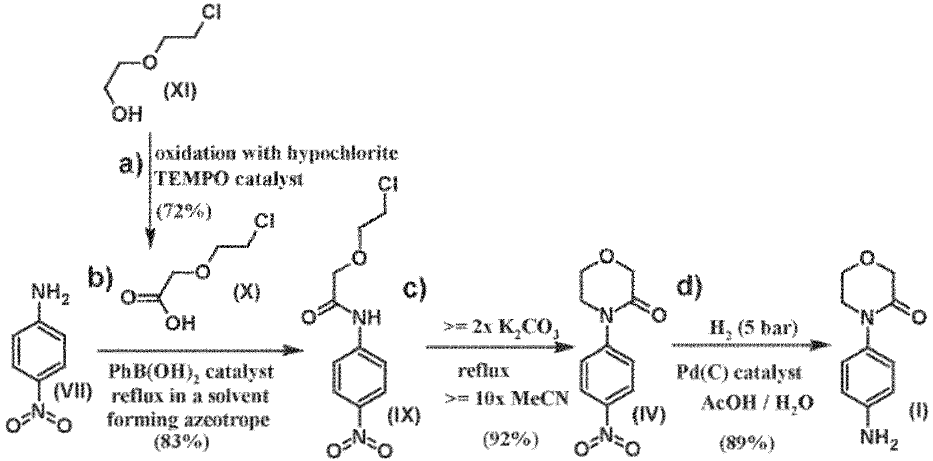

In our procedure 4-nitro-aniline **(VII)** and 2-(2-chloroethoxy)acetic acid **(X)** are coupled in a direct manner in the presence of the catalyst to get 2-(2-chlorethoxy)-N-(4-nitrophenyl)acetamide **(IX).** In this step there is no need for recrystallization, simple "one-pot" reaction is carried out, after the esterification of the catalyst, the product **(IX)** is directly crystallized from the reaction mixture. As a further simplification of the technology, optionally the reaction can be merged with the steps of formation of 2-(2-chloroethoxy)acetic acid **(X).** Similarly "one-pot" reaction can be implemented with merging step-c and step-d. In our procedure dangerous nitration, corrosive and environment polluting compounds (e.g. sulphuric acid, acyl chlorides, evolving hydrogen chloride, chromium trioxide) are avoided.

### DETAILED DESCRIPTION OF THE INVENTION

### Step-a

In our process a 2-(2-chloroethoxy)acetic acid **(X)** is formed from 2-(2-chloroetoxy)ethanol **(XI)** in an oxoammonium-catalyzed oxidation, under phase transfer circumstances. As stochiometric oxidizing agent alkaline metal, alkaline earth metal hypochlorites, advantageously aqueous solutions of sodium- or calcium-hypochlorite are used, and the oxidation is catalysed by 2,2,6,6-Tetramethylpiperidin-1-yl)oxyl (TEMPO) radical.

In one embodiment of the present invention 2-(2-chloroetoxy)ethanol **(XI)** is solved in an aprotic organic solvent, into the intensively stirred reaction mixture the sodium- or calcium-hypochlorite aqueous oxidising agent and 2,2,6,6-Tetramethylpiperidin-1-yl)oxyl catalyst are added, then the two-phase mixture is stirred intensely until oxidation reaction is completed while pH is kept between 8 and 12.

In another embodiment of the present invention the aprotic organic solvent can be selected from aliphatic or aromatic halogenated hydrocarbons, aliphatic or aromatic hydrocarbons, aliphatic ketones, aliphatic ethers, aliphatic esters, aliphatic nitriles, so e.g. the solvent can be dichloromethane, toluene, tetrahydrofuran, methyl isobutyl ketone, ethyl-acetate, acetonitrile, preferably dichloromethane.

Optimally the reaction takes place at a pH in the range 8-12. Beyond this range the catalysis decelerates or even stops. To ensure appropriate pH, either base is added simultaneously with the stoichiometric oxidant or the system is buffered with a weak base at the initial state. As a base alkaline metal and alkaline earth metal hydroxides, hydrogen carbonates, carbonates, hydrogen phosphates, dihydrogen phosphates and phosphates and their aqueous solutions can be used. Preferably, in the initial state the reaction mixture is buffered with sodium hydrogencarbonate.

In the reaction TEMPO catalyst can be used in quantity of 0.1 - 200 mol%, preferably in 0.5 - 2 mol%. Temperature of addition is adjusted between 0-30°C, preferably between 20-25°C. After addition, stirring of the mixture is continued while TEMPO catalyst decomposes the large part of remaining excess of hypochlorite, subsequently the hypochlorite traces are decomposed with a suitable reducing agent, preferably with sodium metabisulfite or sodium sulfite. Reducing agent is used in quantity of 2.5 - 100 mol%, preferably in quantity 2.5 mol%. When the reaction is accomplished, the starting material and the catalyst are washed out from the basic aqueous phase with a suitable solvent non-miscible with water. These solvents can be aliphatic and aromatic halogenated hydrocarbons, aromatic and aliphatic hydrocarbons, aliphatic ketones, aliphatic ethers, aliphatic esters, aliphatic nitriles, e.g. dichloromethane, toluene, tetrahydrofuran, methyl isobutyl ketone, ethyl-acetate, acetonitrile, preferably dichloromethane.

After removing the catalyst and the starting material the aqueous phase is acidified with a suitable organic or inorganic Brønsted acid or with the solution thereof, advantageously with aqueous cc hydrochloric acid.

After the acidification the product is extracted with a water non-miscible solvent. Solvents for extraction can be selected from aliphatic or aromatic halogenated hydrocarbons, aromatic and aliphatic hydrocarbons, aliphatic ketones, aliphatic ethers, aliphatic esters, aliphatic nitriles, e.g. dichloromethane, toluene, xylol, tetrahydrofuran, methyl isobutyl ketone, ethyl-acetate, acetonitrile, advantageously methyl isobutyl ketone. The extracted organic phase is evaporated under reduced pressure, to remove the said solvent, to get the pale yellow oil of 2-(2-chloroethoxy)acetic acid (yield: 71%).

### Step-b

To the oily 2-(2-chloroethoxy)acetic acid **(X)** material obtained in Step-a, 4-nitro-aniline **(VII)** and as catalyst phenylboronic acid are added. In an embodiment of the invention a high boiling point solvent forming aqueous azeotrope, e.g. xylene or toluene, then suitable quantity of 4-nitro-aniline and phenylboronic acid are added. The quantity of 4-nitro-anilline is 0.85-1 equivalent calculating to the formed 2-(2-chloroethoxy)acetic acid, the quantity of added phenylboronic acid is 0.5 - 95 mol%, 5 - 50 mol%, more preferably 25 mol%.

After addition of the starting materials the reaction mixture is heated to reflux temperature, then the formed water is removed by azeotropic distillation from the reaction mixture.

After the reaction is completed, to the mixture alcohol is added for the conversion of the boronic acid catalyst to its corresponding ester, therefore making it soluble in the mother liquor under the crystallisation process. This suitable alcohol can bear one or two hydroxyl groups selected from such aliphatic and aromatic alcohols that, in these reaction circumstances, form toluenemiscible ester with the catalyst. Preferably ethylene-glycol is used.

After the conversion of the catalyst, the reaction mixture is cooled, then the precipitated crystals filtered out, washed with toluene, and dried in vacuum to give the 2-(2-chloroetoxy)-N-(4-nitrophenyl)acetamide **(IX)** as a yellow to light-brown crystalline powder. (Yield 83% calculated to 4-nitro-aniline).

### Step-c

In this ring-closing step the 2-(2-chloroetoxy)-N-(4-nitrophenyl)acetamide **(IX)** product is boiled in acetonitrile with potassium-carbonate, according to the method described in the article of Bioorganic & Medicinal Chemistry Letters 14 (2004) 5817―5822. However, this article alone is not suitable for industrial application, details are not included. We elaborated a simple "one-pot" procedure for the reaction.

At least ten times quantity (ml/g) of acetonitrile and preferably two equivalent potassium-carbonate are used. In such circumstances at the reflux temperature the reaction takes place in 4 hours.

When the reaction is completed, the mixture is cooled back and is acidified with Brønsted acid, advantageously with aqueous hydrochloric acid. Then in a subsequent evaporation the solvent is changed for water, the product is filtered with continuous covering with water.

To accomplish the ring-closing reaction of 2-(2-chloroetoxy)-N-(4-nitrophenyl)acetamide **(IX)** we elaborated another procedure, too. In another embodiment of our invention the 2-(2-chloroetoxy)-N-(4-nitrophenyl)acetamide **(IX)** is refluxed in toluene in the presence of potassium-carbonate and PEG-400 (polyethylene glycol 400).

1-5 equivalents potassium-carbonate quantity was used, optimally two equivalents.

Quantity of PEG-400 was changed between 1 - 100 mol%, at 10 mol% was the optimum.

To accomplish the reaction quickly, temperature of reflux was necessary. At reflux temperature the reaction is completed in two hours.

After the consumption of the starting material, similarly to the case of acetonitrile the reaction mixture is cooled back and is acidified with aqueous hydrochloric acid. Then the solvent is changed for water, the product is washed continuously covering with water to get 4-(4-nitrophenyl)morpholin-3-one as a yellow to light brown crystalline powder **(IV)** (yield: 92%).

### Step-d

The 4-(4-nitrophenyl)morpholin-3-one **(IV)** is solved in aqueous acetic acid then hydrogenated at hydrogen overpressure with palladium on carbon catalyst.

The acetic acid concentration can be 33-100%, preferably 50%. 10-100 times (ml/g) quantity of solvent can be used, at 10 times quantity was the optimum. Changing the palladium content of the palladium-carbon catalyst between 1 and 10%, optimum was 5%. The reaction can be carried out between 15-70°C, optimally in the range of 20-30°C. The overpressure of hydrogen is between 1-5 bar, optimally 1 bar was enough for the complete conversion of the nitro compound **(IV)**. After the consumption of starting material the catalyst filtered off, and the solvent is changed to 2-propanole by distillation, which the product crystallises from.

Then the obtained 4-(4-aminophenyl)morpholin-3-one **(I)** can be used discretionally for the synthesis of rivaroxaban.

Our procedure is economic, the yield of the whole procedure in its optimal form is 68% that exceeds that of the known procedures starting from 2-(2-chloroethoxy)acetic acid.

This procedure to get 4-(4-aminophenyl)morpholin-3-one **(I)** is safe since the nitration is avoided by our synthetic route.

The procedure is an environment protecting one, in contrast to the earlier procedures, it avoids the use of heavy metals and corrosive materials, as chromium trioxide and sulphuric acid, acyl chlorides, or evolving gaseous hydrogen chloride that are dangerous both for the environment and human beings.

The 2-(2-chloroetoxy)-N-(4-nitrophenyl)acetamide **(IX)** is obtained in a direct coupling reaction from 4-nitro-aniline and 2-(2-chloroethoxy)acetic acid **(X)** in the presence of a suitable catalyst. The yield of the boronic acidic procedure known from literature (Sustain Chem. Process (2015) 3:11) is only 50%, in contrast with it in our phenylboronic acidic procedure the achieved yield for the whole procedure is 83%. Moreover in our procedure recrystallization is not necessary, it is a technologically simpler "one-pot" reaction, since after the esterification of the catalyst the product **(IX)** can be crystallised directly from the reaction mixture.

As a further simplification of the technology optionally the Step-b reaction can be merged with Step-a, with forming of 2-(2-chloroethoxy)acetic acid (X).

### EXAMPLES

The following examples are only illustrative ones and are provided to enable one skilled in the art to practice the invention. The examples should not be read as limiting the scope of the invention.

### Example 1:

### 2-(2-chloroethoxy)acetic acid (X)

150 ml dichloromethane, 60 ml tap water, 30.0 ml 2-(2-chloroethoxy)ethanol **(XI)**, 28.52g sodium hydrogencarbonate and 0.22g TEMPO catalyst are measured to a sulfonating flask equipped with condenser, thermometer, dropping funnel. Then at room temperature 355 ml aqueous solution of sodium-hypochlorite is added from the dropping funnel. The mixture is stirred for a further half an hour, then 1.34g sodium metabisulfite is added to the reaction mixture, the two phases are separated in a separation funnel, then the aqueous phase is washed with 2×10 ml dichloromethane. Then the aqueous phase is acidified to pH=1-2 with 34% hydrochloric acid solution. The obtained aqueous solution is extracted with 5x50 ml methyl isobutyl ketone. The combined organic extract is washed with 1×20 ml saturated salt solution, then at a reduced pressure evaporated to constant mass.

The mass of the obtained pale yellow oil: 21.27g, yield: 54%.

### Example 2:

### 2-(2-chloroethoxy)acetic acid (X)

150 ml dichloromethane, 60 ml tap water, 30.0 ml 2-(2-chloroethoxy)ethanol **(XI)**, 28.52g sodium hydrogencarbonate, and 0.22g TEMPO catalyst are measured to a sulfonating flask equipped with condenser, thermometer, dropping funnel. Then at room temperature 240.0 ml solution of 55.62g calcium-hypochlorite in tap water is added from the dropping funnel. The mixture is stirred for a further half an hour, then 1.34g sodium metabisulfite is added to the reaction mixture, the two phases are separated in a separation funnel, then the aqueous phase is washed with 2×10 ml dichloromethane. Then the aqueous phase is acidified to pH=1-2 with 34% hydrochloric acid solution. The obtained aqueous solution is extracted with 5×50 ml methyl isobutyl ketone. The combined organic extract is washed with 1×20 ml saturated salt solution, then at a reduced pressure evaporated to constant mass.

The mass of the obtained pale yellow oil: 28.4g, yield 72%.

### Example 3:

### 2-(2-chloroetoxy)-N-(4-nitrophenyl)acetamide (IX)

90 ml toluene, 10.00g 2-(2-chloroethoxy)acetic acid **(X)** oily extract obtained from one of the previous examples, 8.31g 4-nitro-aniline and 1.84g phenyl-boronic acid are measured into a 125 ml volume sulfonating flask equipped with a Dean-Stark attachment, a condenser and thermometer. The mixture is heated to the reflux temperature (110-111°C), the reaction is checked by a TLC method. After the end of the reaction the mixture is cooled back, 1.00 ml ethylene-glycol is added. Then the mixture is heated again to the temperature of reflux, then it is refluxed for another half an hour. Then the mixture is cooled to a temperature of 0-5°C. The suspension is filtered and washed with toluene. The filtrate is dried to a constant mass. The mass of the obtained pastel yellow powder: 12.98g, yield: 83%.

### Example 4:

### 4-(4-nitrophenyl)morpholin-3-one (IV)

100 ml acetonitrile, 9.35g potassium-carbonate, 10.00g 2-(2-chloroetoxy)-N-(4-nitrophenyl)acetamide **(IX)** are measured into a sulfonating flask equipped with KPG stirrer, N₂ inlet, a condenser dropping funnel and thermometer. The mixture is heated to and kept on reflux temperature until the completion of the reaction (checked by TLC analysis). Then the reaction the mixture is cooled back, a solution of 9.4 ml cc hydrochloric acid and 100 ml tap water is added. Then the suspension is concentrated with evaporation at a reduced pressure until its mass is in the range of 105-115g, then it is cooled back to 20-25°C, then filtered and washed with 2x5 ml tap water. The filtrate is dried at reduced pressure until the mass is constant. The mass of the obtained pastel yellow powder: 7.92g, yield: 92%.

### Example 5:

### 4-(4-aminophenyl)morpholin-3-one (I)

50 ml tap water, 50 ml acetic acid, 10.00g 4-(4-nitrophenyl)morpholin-3-one are measured into a hydrogenating autoclave. The atmosphere of the autoclave is flushed with nitrogen, then 0.50g 10% palladium on carbon is measured, then the atmosphere is changed for hydrogen gas and at 1 bar pressure, and at 20-25°C is started stirring. The stirring is continued until the loss of hydrogen can be observed. Then the reaction mixture is filtered, at a reduced pressure the filtrate is evaporated to a mass of 20-30g, then 100 ml 2-propanole is added, and the suspension obtained is concentrated by evaporation to a mass of 50-60g, then it is cooled to 0-5°C temperature. The precipitated material is filtered off, washed with 2-propanole then dried at a reduced pressure until constant mass. The mass of the obtained white powder: 7.71g, yield: 89%.

## Claims

1. Process for the preparation of **4-(4-aminoophenyl)morpholin-3-one** of Formula **(I)** according to Scheme 6 including the following steps: 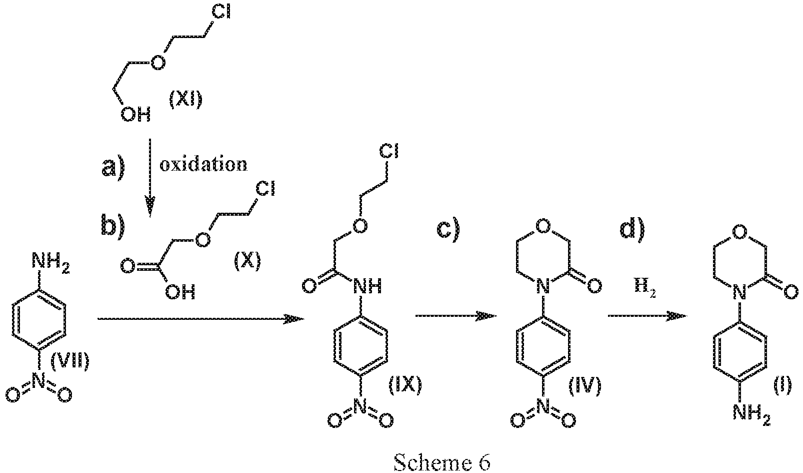
a) 2-(2-chloroethoxy)ethanol of Formula **(XI)** is oxidized to 2-(2-chloroethoxy)acetic acid with an oxidising agent,
b) then the obtained 2-(2-chloroethoxy)acetic acid of Formula **(X)** is reacted with 4-nitro-aniline of Formula **(VII)**,
c) then the obtained 2-(2-chloroetoxy)-N-(4-nitrophenyl)acetamide of Formula (IX) is transformed to 4-(4-nitrophenyl)morpholin-3-one of Formula **(IV)**,
d) then the 4-(4-nitrophenyl)morpholin-3-one of Formula (IV) is hydrogenated to get 4-(4-aminophenyl)morpholin-3-one of Formula **(I)**,
**wherein**
a) in Step-a the oxidising agent is, aqueous sodium- or calcium-hypochlorite and 2,2,6,6-tetramethylpiperidin-1-il)oxyl catalyst is used
b) in Step-b phenylboronic acid catalyst is used,
c) in Step-c the 2-(2-chloroetoxy)-N-(4-nitrophenyl)acetamide **(IX)** product is converted to 4-(4-nitrophenyl)morpholin-3-one **(IV)** in a "one-pot" procedure
d) in Step-d the 4-(4-nitrophenyl)morpholin-3-one **(IV)** is hydrogenated with palladium on carbon catalyst at hydrogen overpressure to get the targeted 4-(4-aminophenyl) morpholin-3-one **(I)** product.

2. Process according to claim 1 wherein in Step-a the 2-(2-chloroethoxy)ethanol is dissolved, the sodium- or calcium-hypochlorite aqueous oxidising agent and 2,2,6,6-tetramethylpiperidin-1-yl)oxyl catalyst are added to the solution, then the two-phase mixture is stirred until oxidation reaction is completed while pH is kept between 8 and 12.

3. Process according to claim 1 wherein in Step-b a solvent forming aqueous azeotrope is added to the oxidised product obtained in Step-a, then 4-nitro-aniline and phenylboronic acid catalyst are added, and the forming water is removed by azeotrope distillation.

4. Process according to claim 1 wherein in Step-c the 2-(2-chloroetoxy)-N-(4-nitrophenyl)acetamide **(IX)** product in at least ten times more (ml/g) quantity of acetonitrile with at least two times more potassium-carbonate is converted to 4-(4-nitrophenyl)morpholin-3-one **(IV).**

5. Process according to claim 2 wherein the quantity of 4-nitro-aniline **(VII)** is 0.85-1 equivalent calculating to the formed 2-(2-chloroethoxy)acetic acid **(X),** and the quantity of added phenylboronic acid is 5 - 50 mol%.

## Patentansprüche

1. Verfahren zur Herstellung von 4-(4-Aminophenyl)morpholin-3-on der Formel (I) gemäß Schema 6, das die folgenden Schritte umfasst: 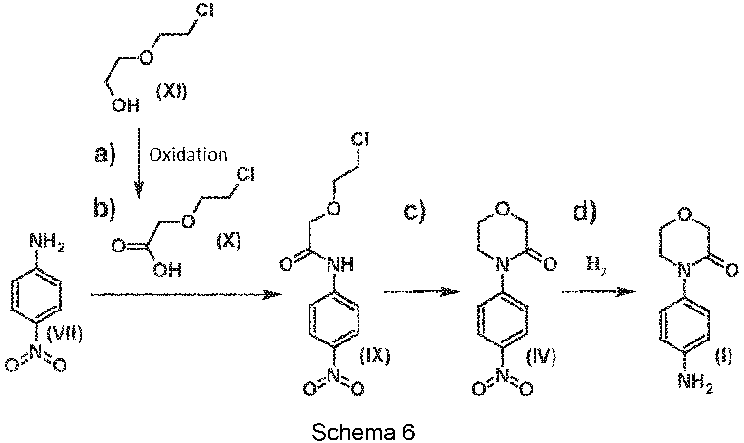
a) 2-(2-Chlorethoxy)ethanol der Formel (XI) wird mit einem Oxidationsmittel zu 2-(2-Chlorethoxy)essigsäure oxidiert,
b) anschließend wird die erhaltene 2-(2-Chlorethoxy)essigsäure der Formel (X) mit 4-Nitroanilin der Formel (VII) umgesetzt,
c) dann wird das erhaltene 2-(2-Chlorethoxy)-N-(4-nitrophenyl)acetamid der Formel (IX) in 4-(4-Nitrophenyl)morpholin-3-on der Formel (IV) umgewandelt,
d) dann wird das 4-(4-Nitrophenyl)morpholin-3-on der Formel (IV) hydriert, um 4-(4-Aminophenyl)morpholin-3-on der Formel (I) zu erhalten,
wobei
a) in Schritt a das Oxidationsmittel wässriges Natrium- oder Calciumhypochlorit ist und (2,2,6,6-Tetramethylpiperidin-1-yl)oxyl-Katalysator verwendet wird,
b) in Schritt b Phenylboronsäure als Katalysator verwendet wird,
c) in Schritt c das Produkt 2-(2-Chlorethoxy)-N-(4-nitrophenyl)acetamid (IX) in einem Eintopfverfahren in 4-(4-Nitrophenyl)morpholin-3-on (IV) umgewandelt wird, und
d) in Schritt d das 4-(4-Nitrophenyl)morpholin-3-on (IV) mit Palladium auf Kohle-Katalysator bei Wasserstoffüberdruck hydriert wird, um das gewünschte Produkt 4-(4-Aminophenyl)morpholin-3-on (I) zu erhalten.

2. Das Verfahren gemäß Anspruch 1, wobei in Schritt a das 2-(2-Chlorethoxy)ethanol gelöst wird, das wässrige Natrium- oder Calciumhypochlorit-Oxidationsmittel und der (2,2,6,6-Tetramethylpiperidin-1-yl)oxyl-Katalysator der Lösung zugesetzt werden, dann das Zweiphasengemisch gerührt wird, bis die Oxidationsreaktion abgeschlossen ist, während der pH-Wert zwischen 8 und 12 gehalten wird.

3. Das Verfahren gemäß Anspruch 1, wobei in Schritt b ein lösungsmittelbildendes wässriges Azeotrop zu dem in Schritt a erhaltenen Oxidationsprodukt hinzugefügt wird, dann 4-Nitroanilin und Phenylboronsäure-Katalysator zugesetzt werden und das entstehende Wasser durch Azeotropdestillation entfernt wird.

4. Das Verfahren gemäß Anspruch 1, wobei in Schritt c das 2-(2-Chlorethoxy)-N-(4-nitrophenyl)acetamid (IX)-Produkt in mindestens der zehnfachen (mL/g) Menge Acetonitril mit mindestens der zweifachen Menge Kaliumcarbonat in 4-(4-Nitrophenyl)morpholin-3-on (IV) umgewandelt wird.

5. Das Verfahren gemäß Anspruch 2, wobei die Menge 4-Nitroanilin (VII) 0,85-1 Äquivalent, bezogen auf die gebildete 2-(2-Chlorethoxy)essigsäure (X), beträgt und die Menge an zugesetzter Phenylboronsäure 5 - 50 Mol-% beträgt.

## Revendications

1. Procédé pour la préparation de la 4-(4-aminophényl)morpholin-3-one de Formule (I) selon le Schéma 6 comportant les étapes suivantes : 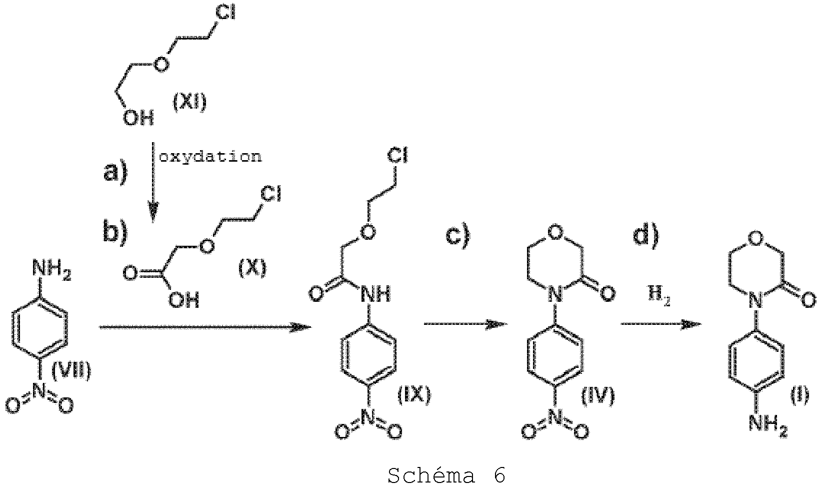
a) le 2-(2-chloroéthoxy)éthanol de Formule (XI) est oxydé en acide 2-(2-chloroéthoxy)acétique avec un agent oxydant,
b) puis, l'acide 2-(2-chloroéthoxy)acétique de Formule (X) est mis à réagir avec de la 4-nitro-aniline de Formule (VII),
c) puis, le 2-(2-chloroéthoxy)-N-(4-nitrophényl)acétamide de Formule (IX) est transformé en 4-(4-nitrophényl)morpholin-3-one de Formule (IV),
d) puis, la 4-(4-nitrophényl)morpholin-3-one de Formule (IV) est hydrogénée pour obtenir la 4-(4-aminophényl)morpholin-3-one de Formule (I),
dans lequel
a) dans l'étape a, l'agent oxydant est l'hypochlorite de sodium ou de calcium aqueux et le catalyseur de 2,2,6,6-tétraméthylpipéridin-1-yl)oxyle est utilisé
b) dans l'étape b, le catalyseur d'acide phénylboronique est utilisé,
c) dans l'étape c le produit de type 2-(2-chloroéthoxy)-N-(4-nitrophényl)acétamide (IX) est converti en 4-(4-nitrophényl)morpholin-3-one (IV) dans une procédure monotope (« one-pot »)
d) dans l'étape d la 4-(4-nitrophényl)morpholin-3-one (IV) est hydrogénée avec un catalyseur de palladium sur carbone avec une surpression d'hydrogène pour obtenir le produit ciblé de type 4-(4-aminophényl)morpholin-3-one (I).

2. Procédé selon la revendication 1, dans lequel dans l'étape a le 2-(2-chloroéthoxy)éthanol est dissous, l'agent oxydant aqueux d'hypochlorite de sodium ou de calcium et le catalyseur de 2,2,6,6-tétraméthylpipéridin-1-yl)oxyle sont ajoutés à la solution, puis le mélange biphasique est agité jusqu'à ce que la réaction d'oxydation soit finie tout en maintenant le pH à une valeur comprise entre 8 et 12.

3. Procédé selon la revendication 1, dans lequel dans l'étape b un solvant formant un azéotrope aqueux est ajouté au produit oxydé obtenu dans l'étape a, puis la 4-nitro-aniline et le catalyseur d'acide phénylboronique sont ajoutés, et l'eau formée est éliminée par distillation azéotropique.

4. Procédé selon la revendication 1, dans lequel dans l'étape c le produit de type 2-(2-chloroéthoxy)-N-(4-nitrophényl)acétamide (IX), dans une quantité au moins dix fois plus importante (ml/g) d'acétonitrile avec au moins deux fois plus de carbonate de potassium, est converti en 4-(4-nitrophényl)morpholin-3-one (IV).

5. Procédé selon la revendication 2, la quantité de 4-nitro-aniline (VII) étant de 0,85 à 1 équivalent calculé par rapport à l'acide 2-(2-chloroéthoxy)acétique (X) formé, et la quantité d'acide phénylboronique ajouté étant de 5 à 50 % en moles.
